Europäisches Patentamt
European Patent Office
Office européen des brevets

(11) Publication number: **0 230 381 A2**

# EUROPEAN PATENT APPLICATION

(21) Application number: 87300332.1

(22) Date of filing: 15.01.87

(51) Int. Cl.4: C 07 C 59/125
C 07 C 69/708
// C07C177/00

(30) Priority: 16.01.86 GB 8600997

(43) Date of publication of application: 29.07.87 Bulletin 87/31

(84) Designated Contracting States: BE CH DE FR GB IT LI NL SE

(71) Applicant: NATIONAL RESEARCH DEVELOPMENT CORPORATION
101 Newington Causeway
London SE1 6BU (GB)

(72) Inventor: Jones, Robert Leslie
62 Caiystane Gardens
Edinburgh EH10 6SY Scotland (GB)

Wilson, Norman Happer
10 Lussielaw Road
Edinburgh EH9 3BX Scotland (GB)

(74) Representative: Stephenson, Gerald Frederick
Patent Department National Research Development Corporation 101 Newington Causeway
London SE1 6BU (GB)

(54) **Prostaglandin synthesis.**

(57) A process for the preparation of a compound of formula (I)

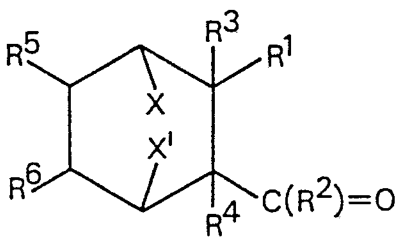

(I)

wherein X and X′ each represents hydrogen or together represent a bridging group $-CH_2-$, $-CH_2-CH_2-$ or -O-; $R^1$ is a 6-carboxyhex-2-enyl group or a modification thereof in which the group is altered by one, or an appropriate combination of two of more, of the following: (a) alteration of the position of the double bond, with the proviso that in the modified group the double bond is not in the alpha-beta position relative to the ring; (b) reduction of the double bond; (c) alteration of the chain length through a decrease of one or two methylene groups or an increase of one to six methylene groups; (d) replacement of one or two methylene groups each separately by an oxygen or sulphur atom, with the proviso that in the modified group no oxygen or sulphur atom is in an alpha position relative to either a doubly bonded carbon atom or to the carboxy group or a derivative thereof and that at least 2 carbon atoms separate any pair of oxygen and/or sulphur atoms; and (e) formation of an amide, ester or salt derivative of the carboxy group; $R^2$ is an aliphatic hydrocarbon group or an aliphatic hydrocarbon group substituted by an aromatic group directly or through an oxygen or sulphur atom; and $R^3$, $R^4$, $R^5$ and $R^6$ are each hydrogen, or $R^3$ and $R^4$ together and/or $R^5$ and $R^6$ together represent the second bond of a carbon-carbon double bond; comprises effecting a Diels Alder reaction between a diene of formula (II) and a dienophile of formula (III)

EP 0 230 381 A2

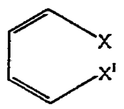

(II)

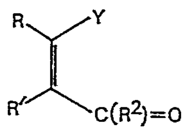

(III)

wherein X, X′ and $R^2$ are as defined for (I); Y is $R^1$ or a precursor therefor in which a double bond is present and/or the terminal group is in different form, either being a free carboxy group rather than the carboxy group derivative present in $R^1$ or being a carboxy group derivative rather than the free carboxy group present in $R^1$; and R and R′ are either each hydrogen or together represent the third bond of a carbon-carbon triple bond; and where appropriate thereafter, in either order, reducing the double bond or bonds in the ring of the Diels Alder adduct and/or converting the group Y to the group $R^1$. The compounds prepared by this process are of value as intermediates for the preparation of biologically active compounds.

**Description**

PROSTAGLANDIN SYNTHESIS

This invention relates to a process for the preparation of compounds exhibiting activity at thromboxane receptor sites and to novel intermediates of use in that process.

Thromboxane $A_2$ ($TXA_2$), which is derived from arachidonic acid via prostaglandin $H_2$ ($PGH_2$), is implicated in several potentially noxious actions on various body systems, including platelet aggregation, bronchoconstriction and pulmonary and systemic vasoconstriction. Thus $TXA_2$ may be involved in the normal sealing of blood vessels following injury but in addition may contribute to pathological intravascular clotting or thrombosis. Moreover, the constrictor actions of $TXA_2$ on bronchiolar, pulmonary vascular and systemic vascular smooth muscle may be important in the development of several anaphylactic conditions including bronchial asthma. There is also some evidence to implicate $PGH_2$ and $TXA_2$ in the genesis of inflammation.

Consequently there is a considerable interest, in the use as pharmaceuticals of compounds having activity at thromboxane receptor sites, especially compounds which are inhibitors of thromboxane activity. In UK Patents 2039909, 2039480, 2081258 and 2113678 compounds having such activity are described which contain a divalent cyclic group carrying a first side chain which is a 6-carboxyhex-2-enyl group or one of certain modifications thereof and a second side chain which may take a number of different forms. The routes described for the preparation of these compounds utilise an intermediate containing the required first side chain or a precursor thereof in which the carboxy group is in a different form and a second side chain which is a formyl group or, for the more preferred type of compound, an acyl group.

The route previously described for the preparation of such acyl group-containing substituents does, however, contain no less than ten separate steps and it is an object of the present invention to provide a novel alternative route which is an improvement upon the previous one for obtaining intermediates of use in the preparation of many of the biologically active compounds described in these four UK patents and of modifications thereof containing a different first side chain.

According to the present invention a process for the preparation of a compound of formula (I)

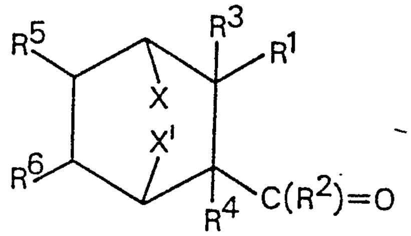

(I)

wherein X and X′ each represents hydrogen or together represent a bridging group $-CH_2-$, $-CH_2-CH_2-$ or -O-; $R^1$ is a 6-carboxyhex-2-enyl group or a modification thereof in which the group is altered by one, or an appropriate combination of two of more, of the following: (a) alteration of the position of the double bond, with the proviso that in the modified group the double bond is not in the alpha-beta position relative to the ring; (b) reduction of the double bond; (c) alteration of the chain length through a decrease of one or two methylene groups or an increase of one to six methylene groups; (d) replacement of one or two methylene groups each separately by an oxygen or sulphur atom, with the proviso that in the modified group no oxygen or sulphur atom is in an alpha position relative to either a doubly bonded carbon atom or to the carboxy group or a derivative thereof and that at least 2 carbon atoms separate any pair of oxygen and/or sulphur atoms; and (e) formation of an amide, ester or salt derivative of the carboxy group; $R^2$ is an aliphatic hydrocarbon group or an aliphatic hydrocarbon group substituted by an aromatic group directly or through an oxygen or sulphur atom; and $R^3$, $R^4$, $R^5$ and $R^6$ are each hydrogen, or $R^3$ and $R^4$ together and/or $R^5$ and $R^6$ together represent the second bond of a carbon-carbon double bond; which comprises effecting a Diels Alder reaction between a diene of formula (II) and a dienophile of formula (III)

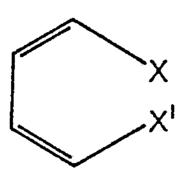

(II)

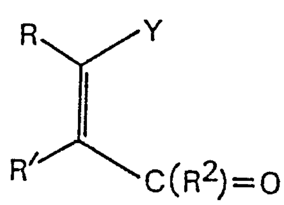

(III)

wherein X, X′ and $R^2$ are as defined for (I); Y is $R^1$ or a precursor therefor in which a double bond is present and/or the terminal group is in different form, either being a free carboxy group rather than the carboxy group

derivative present in $R^1$ or being a carboxy group derivative rather than the free carboxy group present in $R^1$; and R and R′ are either each hydrogen or together represent the third bond of a carbon-carbon triple bond; and where appropriate thereafter, in either order, reducing the double bond or bonds in the ring of the Diels Alder adduct and/or converting the group Y to the group $R^1$.

It will be seen that the modifications of the 6-carboxyhex-2-enyl group which may be made in compounds prepared according to the process of the present invention are of two types. Thus, the modifications either involve the hex-2-enyl group or the 6-carboxy group. Among modifications of the first form, which are listed under (a) to (d) above, certain preferences may be indicated. Thus, if the position of the double bond is altered from the 2,3-position this is more usually to the 5,6-position or more particularly the 4,5-position and especially the 3,4-position. Where the hex-2-enyl group is modified, however, compounds in which the double bond is reduced are of particular interest since, even with a double bond altered from the 2,3-position there may be a chance of the double bond in the group Y of the dienophile (III) shifting into conjunction with the

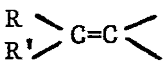

double bond in the course of the reaction thereby forming a diene system with the consequent likelihood of side reactions. Modifications of types (c) and (d) are therefore more often effected together with a modification of type (b). Where the chain length is decreased, this is more often by one methylene group only and where it is increased, this is more often by one to four or particularly one or two methylene groups, an increase in chain length being of generally greater interest than a decrease. A chain of 5 or especially 6 to 8 atoms terminally substituted by a carboxy group or a derivative thereof is therefore generally preferred. When no oxygen or sulphur atom is present a chain of 6 atoms is preferred but in chains containing an oxygen or sulphur atom the position is slightly different as described hereinafter.

The proviso given above under modification (d) precludes the replacement of the methylene group adjacent to the carboxy group but one, or sometimes two, methylene groups at any of the other positions 1 to 5 can each be replaced either by oxygen or sulphur subject to the requirement that those methylene groups are not adjacent to a doubly bonded carbon atom and necessarily that the position is not occupied by a double bond (and that no concomittant decrease in chain length brings the oxygen or sulphur to a position adjacent to the carboxy group). Replacement of the methylene group at the 4-position, although not excluded, is less preferred unless the chain is also extended at the carboxy terminus, since compounds containing a grouping -O (or -S)-$CH_2CH_2$-$CO_2H$ and derivatives thereof are less easy to prepare. Replacement at positions 2 and 3, in conjunction with reduction of the double bond present in the unmodified group, is of some particular interest and, indeed, the replacement of a methylene group at any position is more often effected in conjunction with the reduction of the double bond. Also worth particular mention, however, is replacement of the methylene group at the 5-position. As is also the case with alteration of the double bond to the 4,5-position, such a modification is of especial interest when no modification of type (c) is made involving an increase in chain length at the end of the carbon chain adjacent to the carboxy group (a reduction in chain length involving the methylene group at the 6-position already being precluded). Groups $R^1$ involving a modification of type (d) and optionally also of type (c), together with a modification of type (b), are therefore of some interest, particularly those groups $R^1$ containing oxygen and/or sulphur of the form $-(CH_2)_b-(A)_a-(CH_2)_c-B-CH_2-CO_2H$ and amide, ester and salt derivatives thereof, in which A and B are each separately oxygen or sulphur and either a is 0, b is 0 and c is an integer from 3 to 10, or a is 1, b is 0 or an integer from 1 to 7 and c is an integer from 2 to 9 with the sum of b and c being from 2 to 9. Variations in such a group $R^1$ can involve the $-(CH_2)_b-(A)_a-(CH_2)_c-B-CH_2-$ chain or the terminal $-CO_2H$ group. As regards the chain, it is preferred that a is 0 and that B is oxygen. However, when a is 1, A and B are conveniently each sulphur or, more especially, oxygen. Chains of a total of between 5 and 8 or 10 atoms terminally substituted by a carboxy group or a derivative thereof are of most interest with a preference for chains of at least 6 atoms. Compounds containing a chain of 7 atoms show a generally similar level of activity as compared with those containing a chain of 6 atoms, this being the chain length of the 6-carboxyhex-2-enyl group occurring in natural prostaglandins. There is therefore some interest in chains longer than the natural length. When a is 0, therefore, c is preferably 3, 4, 5, 6 or 7, particularly 4, 5 or 6. When a is 1, b + c is preferably 2, 3, 4, 5 or 6 particularly 3, 4 or 5 and it is also preferred that b is greater than 0 and that c is 2 or 3, the compounds in which a is 1 which are of particular interest being those in which c is 2 and b is 1, 2 or 3, and those in which c is 3 and b is 0 or especially 1 or 2.

As regards the second type of modification of form (e) listed above, the carboxy group derivatives may be (i) esters, especially alkyl esters, for example those containing a $C_1$-$C_{10}$ alkyl group but particularly methyl esters; (ii) amides, which may contain a group $-CONH_2$ or such a group in which the nitrogen atom is substituted, especially by one or two groups selected from substituted or unsubstituted phenyl groups, for example as described hereinafter, alkyl groups, for example $C_1$-$C_{10}$ alkyl groups, and from more complex groups such as $-SO_2CH_3$ or an analogue thereof containing a $C_2$-$C_{10}$ alkyl group, for example to provide a group of the form $-CONHSO_2CH_3$; and (iii) salts, particularly containing one of various physiologically acceptable cations. Salt derivatives are of especial interest, specific examples of salts being those formed with an alkali metal such as sodium or with quaternary ammonium ions or amines such as tris (the symbol tris represents the compound 2-amino-2-hydroxy-methylpropane 1,3-diol). It will be appreciated that many of such

compounds containing a modification of form (e) are in fact bioprecursors for the corresponding compound containing a carboxy group.

Examples of specific groups $R^1$ are $-CH_2-CH=CH-(CH_2)_3-CO_2H$, especially $-(CH_2)_6-CO_2H$ and particularly $-(CH_2)_2O(CH_2)_3CO_2H$, $-(CH_2)_4OCH_2CO_2H$, $-(CH_2)_5OCH_2CO_2H$, $-(CH_2)_6OCH_2CO_2H$, $-CH_2O(CH_2)_2OCH_2CO_2H$, $-(CH_2)_3O(CH_2)_2OCH_2CO_2H$, $-(CH_2)_2O(CH_2)_3OCH_2CO_2H$, $-(CH_2)_2O(CH_2)_2OCH_2CO_2H$ and $-CH_2O(CH_2)_3OCH_2CO_2H$, as well as amide, ester and salt derivatives of these groups.

As indicated, $R^2$ is a hydrocarbon group or such a group substituted by an aromatic group either directly or through an oxygen or sulphur atom, the preference in both cases being for an acyclic hydrocarbon group. Unsubstituted hydrocarbon groups $R^2$ may conveniently be of one to five, six, seven, eight, nine, ten or even more carbon atoms, being for example a branched or unbranched alkyl group such as methyl, ethyl, propyl, butyl, amyl, etc. Substituted aliphatic hydrocarbon groups may conveniently be formed from similar alkyl groups, preferably those containing 3 carbon atoms, particularly 2 carbon atoms and especially 1 carbon atom. Preferred substituted aliphatic hydrocarbon groups $R^2$ contain unbranched alkylene groups such as methylene, ethylene and propylene.

The aromatic groups present in substituted aliphatic hydrocarbon groups $R^2$ may be hydrocarbon or heterocyclic groups which may be unsubstituted or substituted. Moreover, the term 'aromatic group' as used herein extends to groups derived from ring systems having aromatic properties but in which the $\pi$-electron system is not fully delocalised over the entire ring system, such groups including those derived from fluorene, and the dibenzocyclohexanes and dibenzocycloheptanes, such as 1,2,4,5-dibenzocyclohexane and 1,2,4,5-dibenzocycloheptane, and from dihydrobenzoxazole, dihydrobenzthiazole, N-methyldihydrobenzoxazole and N-methyldihydrobenzthiazole. The heterocyclic groups may conveniently contain one, two or more similar or different nitrogen, oxygen or sulphur atoms and, in addition to the groups just described, may include groups derived from pyridyl, benzthiazole and benzoxazole. Of particular interest are heterocyclic groups which are linked to the aliphatic hydrocarbon group through a hetero atom, such as pyrid-1-yl.

Among the aromatic groups contained in groups $R^2$, aromatic hydrocarbon groups, for example naphthyl (including both napth-1-yl and napth-2-yl) and particularly phenyl are however generally of rather greater interest than heterocyclic groups. Both the aromatic hydrocarbon and the heterocyclic groups may be substituted, by one or more of various types of substituent, particularly by alkoxy groups, for example those containing alkyl groups of one, two, three or more carbon atoms as described above and especially methoxy, and by substituents being or containing a halogen residue, for example halogeno groups such as bromo, chloro and especially fluoro, and also halogeno-substituted alkyl groups such as $CF_3$. Examples of other substituents are sulphamoyl groups which may optionally be N-substituted, amino groups which may be free or substituted, for example dimethylamino, hydroxyl, nitro and alkyl groups, for example of 1 to 3 carbon atoms or otherwise as described above, etc.

In the case of some substituents, for example nitro, substitution by a single substituent may be of particular interest. In general, however, substitution may be present at one or more of the ortho, meta and para positions of a phenyl ring or at a combination of two or more such positions (including two ortho or two meta positions), for example at the 2- and 3- or 2-and 4-positions.

In general, groups $R^2$ being either an aliphatic hydrocarbon group substituted directly by an aromatic group or particularly an unsubstituted aliphatic hydrocarbon group are of most interest. Where $R^2$ is such a substituted group, then it is preferred that the aromatic group is not attached to a carbon atom of the aliphatic group which is itself attached directly to the carbon atom of the group $C(R^2)=0$. Thus, for example, a 2-phenylethyl group is preferred to a 1-phenylethyl or phenylmethyl (benzyl) group. The size of the group $R^2$ can however influence the ease with which the compounds may be prepared and $R^2$ is preferably one of the smaller alkyl groups, for example of 1 to 3 carbon atoms, in substituted form, or particularly in unsubstituted form, for example ethyl and especially methyl.

Among the various ring systems present in compounds (I) which may be prepared according to the process of the present invention the bridged ring systems are of particular interest, especially those in which $R^3$ and $R^4$ are each hydrogen, i.e. the bicyclo [2,2,1] heptane, bicyclo [2,2,1] hept-2Z-ene, bicyclo [2,2,2] octane, bicyclo [2,2,2] oct-2Z-ene, 7-oxa-bicyclo [2,2,1] heptane and 7-oxa-bicyclo [2,2,1] hept-2Z-ene ring systems.

It will be appreciated that the structures of the compounds (I) described above provide various opportunities for the occurrence of stereoisomerism. In the case of the saturated and the mono-unsaturated ring systems the substituent groups $R^1$ and $C(R^2)=0$ may be in the cis or trans relationship to each other, compounds of the latter configuration more generally being preferred although cis compounds (particularly in the 5-exo, 6-exo rather than the 5-endo form, as referred to below, where appropriate) are not without interest, particularly in the case of the cyclohexane, cyclohexene and the two oxygen bridge ring systems. Moreover, in most cases different isomers will exist which vary according to the way in which the substituent groups $R^1$ and $C(R^2)=0$ are disposed in relation to the bridging group provided by X and X'.

The present invention can be used to prepare compounds (I) in the various stereoisomeric forms in which they exist, the two possible trans forms of the bridged ring systems being illustrated below in one of the two enantiomorphic forms which can exist, the other enantiomorph having a structure which is the mirror image of that shown. The mono-unsaturated bridged ring system ($R^3$ and $R^4$ = H, $R^5$ and $R^6$ = second bond of $>C=C<$) is illustrated, the symbol D in this case being limited to a bridging group $-CH_2-$, $-CH_2CH_2-$ or $-O-$. (The cis isomers can of course also generally exist in two forms for these same ring systems, i.e. the 5-exo, 6-exo and 5-endo, 6-endo forms, both of which can exist as two enantiomers.)

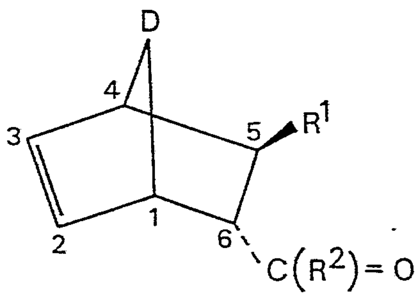

5-exo, 6-endo

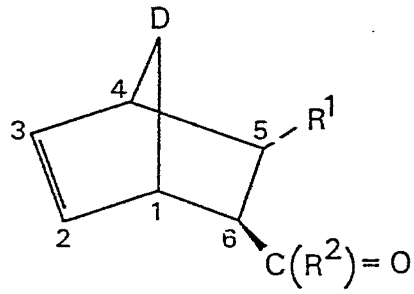

5-endo, 6-exo

It will be seen that the bridged ring system is shown in a three dimensional representation and that the numbering used herein for the various positions of the ring is indicated, the group X being position 7 or positions 7 and 8. It should be noted that the system of numbering adopted is chosen so that the double bond in the mono-unsaturated bridged ring system receives the lowest number possible (2), the substituents $R^1$ and $C(R^2)=O$ then being at the 5 and 6 positions respectively. For conformity a similar system of numbering is followed for the analogous di-unsaturated and saturated bridged ring systems. The di-unsaturated bridged ring systems can of course exist only in the cis form at the 5,6 positions but the saturated systems can in general exist in similar stereoisomeric forms to the mono-unsaturated systems, with the exception of the bicyclo [2,2,2] octane system which possesses a greater degree of symmetry than the other two ring systems as the two bridging groups attached together at the bridge positions (1 and 4) are identical, both being $-CH_2CH_2-$. In the case of this one bridged ring system, therefore, the position is similar to that for the cyclohexane and cyclohexene ring systems and the preferred trans isomer does not show the endo, exo type of isomerism which can occur with all of the other bridged ring systems. For the other saturated bridged ring systems and all of the mono-unsaturated systems, where two trans forms can exist, the preference is generally for compounds of the 5-endo, 6-exo form although the 5-exo, 6-endo form is not without interest, and in the case where D is -O- may be of equal or greater interest. Where the substituent $R^1$ is a 6-carboxyhex-2-enyl group or a group modified therefrom but still containing the double bond, then the configuration about this bond is preferably cis (Z) rather than trans (E). In addition to the foregoing isomerism, as indicated previously the compounds which may be prepared by the process of the present invention will additionally be resolvable into enantiomorphic forms.

As has been indicated hereinbefore, the present invention relates not only to the preparation by an improved route of intermediates already described in the four UK patents previously mentioned but also the preparation of other intermediate compounds (I) which have not previously been described in the literature. The present invention also extends to such novel compounds (I) and in particular to (i) compounds of formula (I) in which $R^3$ and $R^4$ together represent the second bond of a carbon-carbon double bond, the other symbols being as defined hereinbefore, and (ii) compounds of formula (I) in which $R^1$ involves a modification (a) in which the double bond of the 6-carboxyhex-2-enyl group is altered to the 4,5 or 5,6 position, a modification (c) involving an increase of three to six methylene groups, or a modification (d) in which the methylene group at the 4 or particularly the 5 position of the 6-carboxyhex-2-enyl group is replaced by an oxygen or sulphur atom or in which any two methylene groups are replaced, in each case optionally together with one or more of the other forms of modification which are appropriate, the symbols other than $R^1$ being as defined hereinbefore.

Examples of specific compounds (I) which may be prepared by the process of the present invention, including compounds which are novel <u>per se</u> are those of formula (Ia)

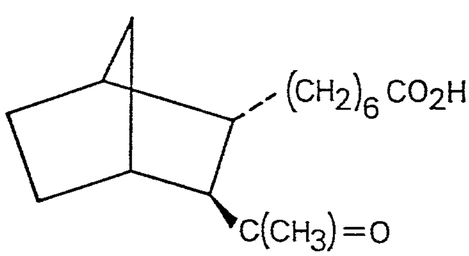

(Ia)

and related compounds in which one or more of the following variations is present: (a) the converse trans stereochemical arrangement of the substituents $R^1$ and $C(CH_3)=O$; (b) one of the alternative saturated or mono-unsaturated bridged groups described hereinbefore in place of the bicyclo [2,2,1] heptane group, for example a bicyclo [2,2,2] octane group; (c) a 7-carboxy-6-oxaheptyl or 6-carboxy-5-oxahexyl group in place of the 6-carboxyhexyl group; (d) derivatisation of the carboxy group as an amide, ester or salt; (e) an ethyl group

in place of the methyl group.

The Diels Alder reaction leading to the compound (I) may be effected by various of the procedures described in the art for this class of reaction but it has been found that a considerable advantage accrues from the use of a Lewis acid catalyst, for example aluminium chloride or more preferably titanium tetrachloride, stannic chloride or especially boron trifluoride. It is still also preferred, however, to carry out the reaction at an elevated temperature, for example at between 60-120°C, especially 80-100°C. When using the Lewis acid catalyst it can be advantageous to add the diene to the dienophile and the Lewis acid, thereby maintaining an excess of the dienophile in the reaction mixture.

Where the desired compound (I) contains two ring double bonds then it may be obtained directly, subject to any necessary modification of the group Y, from a dienophile (III) in which R and R′ together represent the third bond of a carbon-carbon triple bond, and when it contains a single ring double bond it may conveniently similarly be obtained directly from a dienophile (III) in which R and R′ are each hydrogen. When the bridged ring is saturated, either type of dienophile may be used followed by the reduction of the double bond or bonds present in the Diels Alder adduct, such a reduction conveniently taking the form of a catalytic hydrogenation, for example using particularly palladium on carbon or, when the chain of the group $R^1$ contains an oxygen or sulphur atom, a ruthenium catalyst such as ruthenium on alumina or carbon or a rhodium catalyst, particularly Wilkinson's catalyst. Since a selective reduction would be required in order to produce a compound containing a double bond in the group $R^1$ and a saturated ring system, such compounds may often be more conveniently prepared by the procedures described in the earlier patents.

Where the desired compound (I) contains a group $R^1$ terminating in a free carboxy group or a salt derivative thereof, it is often more convenient to employ a dienophile (II) in which the group Y contains a carboxy group in protected form, the carboxy group being deprotected after the Diels Alder reaction and any subsequent reduction. In particular the group Y may terminate in an ester group, for example the ethyl ester, and this may finally be removed using basic hydrolysis conditions to give the free carboxy group which can then be converted to salt form using the appropriate base if so desired. Compounds (I) prepared according to the process of the invention and containing a free carboxy group can of course also be converted to amide or ester form if desired, conventional procedures being used and, indeed, compounds containing an oxygen or sulphur atom in the group $R^1$ in a position beta to the carboxy group may often particularly readily undergo ester formation, for example on solution in an alcohol such as ethanol.

The selection of a dienophile (III) in which R and R′ are hydrogen of either cis or trans stereochemistry about the double bond will dictate the cis or trans stereochemistry at the 5 and 6 positions in the resulting Diels Alder adduct but it will be appreciated that a mixture of isomers will still result which, as explained hereinbefore, vary in the orientation of the groups $R^1$ and $C(R^2)=0$ relative to the bridging group X. In general, dienophiles of a trans configuration are most readily accessible and will lead to a mixture of the 5-exo, 6-endo and 5-endo, 6-exo isomers of many of the compounds (I), as discussed hereinbefore, which can be separated if so desired. However, the necessity for such a separation does not occur in the case of the cyclohexene and cyclohexane ring systems and of one particular bridged ring system, the bicyclo [2,2,2] octane ring system, in view of the symmetrical nature of these systems. In the case of the cyclohexene ring system an adduct in the form of a sole cis or trans isomer will result from the use of the cis or trans dienophile (III), the same situation pertaining in the corresponding cyclohexane which is obtained on reduction. In the case of the bicyclo [2,2,2] octane ring system reduction of the mixed isomer adduct containing a bicyclo [2,2,2] oct-2Z-ene ring system will yield a sole cis or trans isomer depending on the stereochemistry of the original dienophile (III). The process of the present invention is thus particularly adapted to the preparation of these ring systems and particularly the bridged bicyclo [2,2,2] octane compounds, especially those not containing a carbon-carbon double bond in the group $R^1$ and, for example, containing a group $R^1$ with a modification of type (d) and optionally also of type (c). It will be appreciated that compounds containing this ring system, as with the compounds containing the other ring systems, can exist in different enantiomeric forms, the single enantiomers in general being obtainable by the resolution of a pair of enantiomers.

The dienes (II) used in the process of the present invention are readily available commercially, being buta-1,3-diene, cyclopentadiene, cyclobuta-1,3-diene and furan. In contrast, the dienophiles (II) will usually need to be prepared but are obtainable by relatively simple procedures, as illustrated below for the preparation of the dienophile (III) containing a group $R^1$ with a modification of type (d), in particular a 6-carboxy-5-oxahexyl group $R^1$ protected as the ethyl ester derivative, a methyl group $R^2$ and a double rather than a triple bond.

$$HO\text{-}CH_2(CH_2)_3CH_2\text{-}OH \xrightarrow[\text{DMF}]{\text{(1) NaH} \quad \text{(2) } BrCH_2CO_2Et} HO\text{-}CH_2(CH_2)_3CH_2OCH_2CO_2Et$$

$$\xrightarrow{DMSO/TFAA/Et_3N} CHO\text{-}(CH_2)_3CH_2OCH_2\text{-}CO_2Et \xrightarrow{NaH/(MeO)_2POCH_2COCH_3}$$

$$CH_3CO\text{-}CH{=}CH\text{-}(CH_2)_3CH_2OCH_2\text{-}CO_2Et.$$

(In this reaction scheme conventional abbreviations are used in designating the reactants and solvents, i.e. Me : methyl; Et : ethyl; DMF : dimethylformamide; DMSO : dimethylsulphoxide; TFAA : trifluoroacetic acid anhydride.)

As will be apparent to those skilled in the art, variations of this procedure may be used for the preparation of the same dienophile (III) and more particularly for the preparation of alternative dienophiles (III). Thus, in particular, the phosphonate reagent used in the Horner reaction which is the last step of the procedure given above may be varied through replacing the terminal acetyl group by an alternative acyl group in order to produce dienophiles containing an alternative group $R^2$.

A further form of variation of the procedure shown involves the use of a different compound in the Horner reaction with the phosphonate reagent. Thus, it will be apparent that a diol of different chain length may be used to provide a different group $R^1$ with a modification of type (d) and also of type (c), so that, for example, the diol $HO\text{-}CH_2\text{-}(CH_2)_4CH_2\text{-}OH$ will provide a dienophile $CH_3CO\text{-}CH{=}CH\text{-}(CH_2)_4CH_2OCH_2\text{-}CO_2Et$. It has been found that the reaction of the diol $HO\text{-}CH_2(CH_2)_3CH_2\text{-}OH$ with ethyl bromoacetate leads to a mixture of the desired compound $HO\text{-}CH_2(CH_2)_3CH_2OCH_2CO_2Et$ and of the bromo compound $HO\text{-}CH_2(CH_2)_3CH_2O\text{-}COCH_2Br$. It is possible to treat this mixture with dihydropyran to form the respective tetrahydropyranyl ethers, then to hydrolyse the respective ester groups of these ethers and separate the two products by $Et_2O$ extraction from an aqueous basic medium. The two separate ethers may then be reacted to remove the tetrahydropyranyl ether function and generate the appropriate functional group at the other end of each molecule. This procedure, which provides an alternative to the difficult separation of the compounds $HO\text{-}CH_2(CH_2)_3CH_2OCH_2CO_2Et$ and $HO\text{-}CH_2(CH_2)_3CH_2OCOCH_2Br$, is illustrated below for the generalised use of a diol $HO\text{-}(CH_2)_r\text{-}OH$ in which r is an appropriate integer and THPO is tetrahydropyranyloxy.

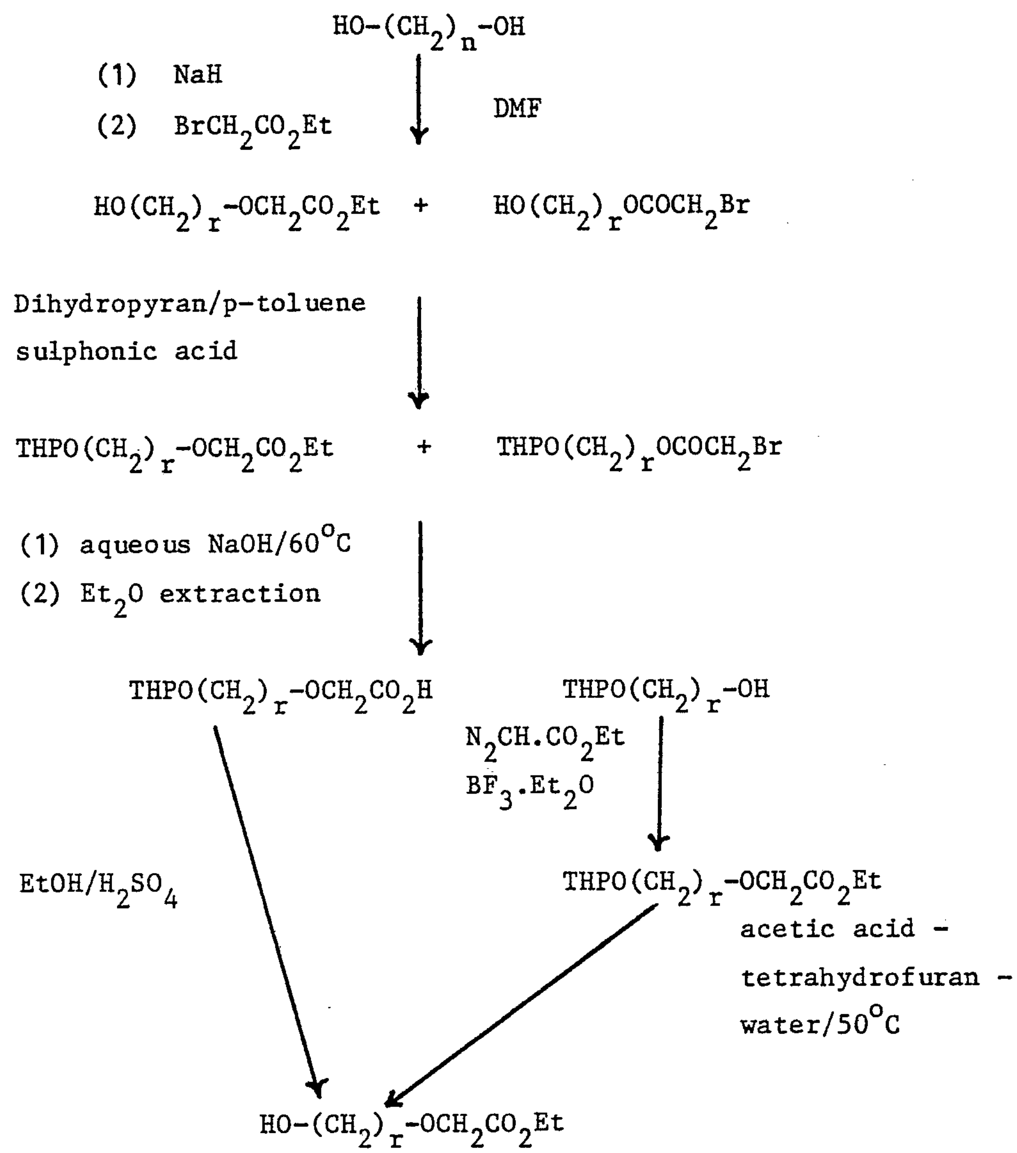


Furthermore, the schemes (1) and (2) illustrated below exemplify the production of dienophiles suitable for the preparation of compounds (I) containing either two hetero atoms in the chain of the group $R^1$ or solely carbon atoms in that chain.

(1) 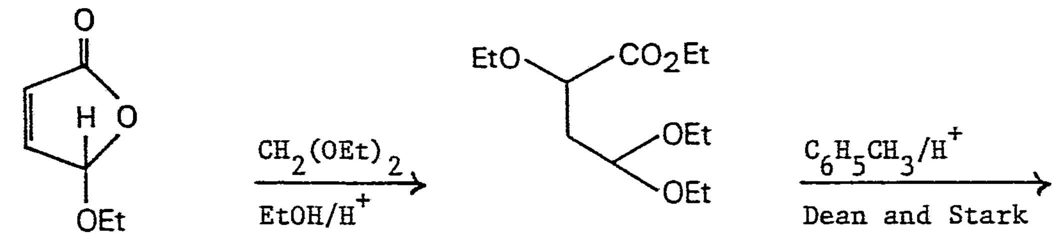


$(EtO)_2$-CH=CH-$CO_2$Et $\xrightarrow{iBu_2AlH}$ $(EtO)_2$CH=CH-$CH_2$OH

$\xrightarrow{\overset{O}{CH_2CH_2}/OH^-}$ $(EtO)_2$-CH=CH-$CH_2$O-$CH_2CH_2$OH $\xrightarrow{Base/BrCH_2CO_2Et}$

$(EtO)_2$-CH=CH-$CH_2$O-$CH_2CH_2OCH_2CO_2$Et $\xrightarrow{H^+/H_2O}$

CHO-CH=CH-$CH_2$O-$CH_2CH_2OCH_2CO_2$Et $\longrightarrow$

$R^2$CO-CH=CH-$CH_2$O-$CH_2CH_2OCH_2CO_2$Et.

(In a reaction such as that with $(EtO)_2$-CH=CH-$CH_2$O-$CH_2CH_2$OH where only one reactive functional group is present, the ethyl bromoacetate may conveniently be replaced by ethyl diazoacetate.)

The replacement of the formyl group by a group $R^2$CO- in the last reaction may be effected in several ways, the formyl group conveniently being oxidised to a carboxy group which is then converted to the acid chloride, which is in turn converted to an acyl group, for example by reaction with the appropriate cadmium compound $(R^2)_2$Cd, dimethyl cadmium providing an acetyl group. The procedure can be varied further through chain extension of the compound $(EtO)_2$-CH=CH-$CH_2$OH, for example via the corresponding nitrile, before the reaction with ethylene oxide. Thus, for example, a single chain extension will give an aldehyde/ester $R^2$CO-CH=CH-$CH_2CH_2$-O-$CH_2CH_2OCH_2CO_2$Et.

(2)

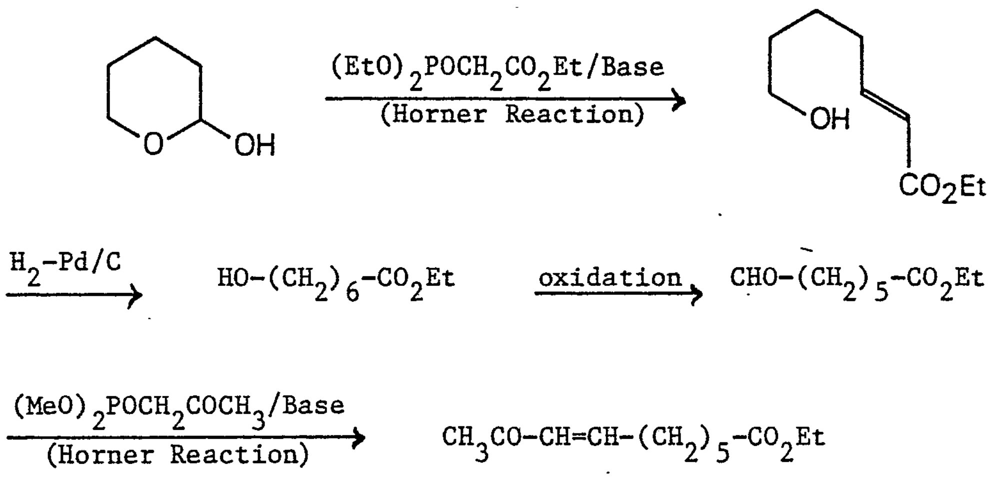


The corresponding compounds containing a 5- or 7-membered ring system may be used in place of the 2-hydroxytetrahydropyran as a starting material to provide dienophiles $CH_3$-CO-CH=CH-$(CH_2)_4$-$CO_2$Et and $CH_3$CO-CH=CH-$(CH_2)_6$-$CO_2$Et.

In a further aspect of the present invention an exactly similar process is applied to the preparation of compounds (I) in which $R^2$ is hydrogen. The present invention thus also extends to those compounds, per se, corresponding to the novel compounds referred to previously but in which $R^2$ is hdyrogen rather than an aliphatic hydrocarbon group or an aliphatic hydrocarbon group substituted by an aromatic group directly or through an oxygen or sulphur atom. Examples of such specific compounds having a formula (I) in which $R^2$ is hydrogen include the compound having the formula (Ia) but with the group -C($CH_3$)=0 replaced by -CHO and related compounds in which one or more of the variations (a), (b), (c) and (d) indicated hereinbefore in relation to formula (Ia) is present.

Compounds (I) according to the present invention, whether old or novel, may be used in preparing biologically active compounds in an exactly similar way to that described in the four earlier patents (corresponding patents and patent applications are for UK Patent 2039909, US Patent 4430345 and Japanese Patent Application 80/500131; for UK Patent 2039480, US Patent 4438136 and Japanese Patent Application 80/500132; for UK Patent 2081258, US Patent 4596823 and Japanese Patent Application 81/502230; and for UK Patent 2113678, US Patent Application 531899 and Japanese Patent Application 83/500327). In addition to their use in the preparation of biologically active compounds containing a second side chain as described in these patents the compounds (I) of the present invention may also be used in the preparation of related compounds containing alternative forms of second side chain, for example compounds such as those described in UK Patent Application 2119375 (corresponding US Patent Application 374125) and European Patent Applications 0094792 and 0107995 (corresponding US Patent Applications 378560 and 436741, respectively) and modifications thereof. Various of the novel, biologically active compounds which may be prepared using compounds (I) obtained by the process of the present invention are the subject of a UK patent application in our names of even date herewith.

The invention is illustrated by the following Examples.

## EXAMPLES

### Example 1

5-endo-(6′-Ethoxycarbonyl-5′-oxahexyl)-6-exo-acetyl-bicyclo [2,2,2] oct-2Z-ene and 5-exo-(6′-ethoxycarbonyl-5′-oxahexyl)-6-endo-acetyl-bicyclo [2,2,2] oct-2Z-ene

(1) Ethyl 8-hydroxy-3-oxa-octanoate

A solution of 1,5-pentanediol (50 g, 480 mmol) in 100 ml of dry, redistilled dimethylformamide (DMF), is added dropwise, under nitrogen, to sodium hydride (11.3 g, 480 mmol) in 300 ml DMF (obtained from 19.2 g of a 60% w/v dispersion of sodium hydride in oil by washing with sodium dried petroleum spirit). After the addition is complete (about 45 minutes) the reaction mixture is heated to about 70° C and stirring is continued for a further 4 hours when all hydrogen evolution has ceased. The reaction mixture is then cooled to 0° C and ethyl bromoacetate (80.2 g; 480 mmol) is added, the ester being added in one portion in order to minimise ester cleavage by the basic reaction medium. After cessation of the consequent vigorous reaction, the mixture is heated at about 70° C for a further 2 to 3 hours before the removal of the majority of the solvent by vacuum distillation. On cooling, the residue is poured into water and the product is isolated by ethyl acetate extraction and purified by chromatography on Florisil to provide the title compound as an oil (61.2 g, 67%), $\nu_{max}$ (film):

3450, 1740, 1195, 1130 $cm^{-1}$.

A good purification of this product is not easy to achieve in view of the contamination by the compound $HO-(CH_2)_5OCOCH_2Br$. In a variation of this procedure the mixed products from the reaction with the ethylbromoacetate may each be converted via their tetrahydropyranyl ethers to the title compound by the procedure described and illustrated in the main text.

(2) Ethyl 7-formyl-3-oxaheptanoate

To a solution of dimethylsulphoxide (18.6 ml, 263 mmol) in 75 ml of $CH_2Cl_2$ at -60°C, under nitrogen, is added dropwise a solution of trifluoroacetic anhydride (27.8 ml, 197 mmol) in 75 ml of $CH_2Cl_2$, over about 15 minutes. The reaction mixture is stirred for a further 15 minutes at -60°C and the hydroxy/ester (1) (25 g, 132 mmol) in 150 ml $CH_2Cl_2$ is then added at such a rate as to keep the reaction temperature below -60°C (over about 30 minutes). After a further 15 minutes at this temperature, triethylamine (55 ml, 395 mmol) is added slowly over about 15 minutes, the reaction mixture is allowed to warm to ambient temperature (about 1.5 hours) and is then quenched with water. The product is isolated by extraction with dichloromethane and purified by chromatography on Florisil to provide the title compound as an oil (20.7 g, 84%), $\nu_{max}$ (film) 1750, 1725, 1195, 1130 $cm^{-1}$.

(3) Ethyl 10-oxo-8(E)-3-oxa-undecenoate

A solution of dimethyl 2-oxopropyl-phosphonate (10.6 g, 64 mmol) in 50 ml of dry, redistilled tetrahydrofuran (THF) is added over a period of about 20 minutes to a vigorously stirred suspension of sodium hydride (1.5 g, 64 mmol) in THF (obtained from 2.5 g of a 60% w/v dispersion of sodium hydride in oil by washing with sodium dried petroleum spirit), under nitrogen. The resulting suspension of a flocculant precipitate is stirred for a further 1 hour and the aldehyde/ester (2) (10 g, 53 mmol) in 50 ml THF is then added dropwise. The reaction mixture is stirred for a further 3 to 4 hours at room temperature before quenching with a 1% v/v aqueous solution of acetic acid. The product is isolated by ether extraction and purified by chromatography on silicic acid, the desired fraction being eluted in toluene/ethyl acetate (90:10 v/v), to provide the title compound as an oil (7.5 g, 62%), $\nu_{max}$ (film) 1745, 1670, 1620, 1190, 1125 $cm^{-1}$, $\lambda_{max}$ (MeOH) 222 nm, $\varepsilon_{max}$ 3488.

(4) 5-endo-(6′-Ethoxycarbonyl-5′-oxahexyl)-6-exo-acetyl-bicyclo [2,2,2] oct-2Z-ene and 5-exo-(6′-ethoxycarbonyl-5′-oxahexyl)-6-endo-acetyl-bicyclo [2,2,2] oct-2Z-ene

To a solution of the enone/ester (3) (2.5 g, 11 mmol) in 50 ml of sodium dried benzene is added a catalytic amount of boron trifluoride etherate (0.27 ml; 2.2 mmol), followed by cyclohexa-1,3-diene (2.1 ml; 22 mmol). The resulting solution is refluxed under nitrogen for 4 to 5 hours and, on cooling, the mixture is poured onto a saturated aqueous solution of sodium hydrogen carbonate. The product is isolated by ether extraction and purified by chromatography on Florisil, the desired fraction being eluted in toluene/ethyl acetate (95:5 v/v), to yield the mixture of title compounds as an oil (1.9 g, 56%), $\nu_{max}$ (film) 1750, 1705, 1195, 1130, 700 $cm^{-1}$, $\delta(CDCl_3)$ 6.35 (1H, dd), 6.00 (1H, dd), 4.16 (2H, q), 4.00 (2H, s), 3.51 (2H, t), 2.75 (1H, m), 2.4 (1H, m), 2.08 (3H, s), 1.92-1.05 (12H, m), 1.28 (3H, t).

Example 2

trans-5-(6′-Carboxy-5′-oxahexyl)-6-acetyl-bicyclo [2,2,2] octane

(1) trans-5-(6′-Ethoxycarbonyl-5′-oxahexyl)-6-acetyl-bicyclo [2,2,2] octane

The mixture of 5-endo-(6′-ethoxycarbonyl-5′-oxahexyl)-6-exo-acetyl-bicyclo [2,2,2] oct-2Z-ene and 5-exo-(6′-ethoxycarbonyl-5′-oxahexyl)-6-endo-acetyl-bicyclo [2,2,2] oct-2Z-ene (2.0 g, 65 mmol, prepared as in Example 1), is dissolved in ethanol and a catalytic amount of palladium on activated charcoal (10% w/w) is added. The resulting mixture is vigorously stirred under an atmosphere of hydrogen until the uptake of hydrogen is complete. The catalyst is removed by filtration through a Celite plug and the filtrate evaporated to give, as an oil, the title compound in admixture with trans-5-(4′-hydroxy)-6-acetyl-bicyclo [2,2,2] octane in a ratio of 55:45 w/w ester:alcohol[1].

(2) trans-5-(6′-Carboxy-5′-oxahexyl)-6-acetyl-bicyclo [2,2,2] octane

The mixture from (1) is dissolved in dioxan/water (1:1 v/v) and an aqueous solution of 2M NaOH is added to give a final base concentration of N/20. The reaction mixture is heated for 2.5 hours at about 60°C then cooled and poured into water. The aqueous phase is washed with ether to remove the alcohol component of the starting material and is then acidified to pH 3-4 and the desired product isolated by ether extraction. The extract is purified by chromatography on silicic acid, the desired fraction being eluted in toluene/ethyl acetate (85:15 v/v), to yield the title compound as an oil (1.9, 56%), $\nu_{max}$ (film) 1750, 1705, 1195, 1130, 700 $cm^{-1}$, $\delta(CDCl_3)$ 6.35 (1H, dd), 6.00 (1H, dd), 4.16 (2H, q), 4.00 (2H, s), 3.51 (2H, t), 2.75 (1H, m), 2.4 (1H, m), 2.08 (3H, s), 1.92-1.05 (12H, m), 1.28 (3H, t).

Example 3

[1] Better catalytic selectivity in favour of the ester is achieved by the use of Wilkinson's catalyst or ruthenium on carbon.

trans-5-(7′-Carboxy-6′-oxaheptyl)-6-acetyl-bicyclo [2,2,2] octane

The procedure of Examples 1 and 2 is followed but commencing with 1,6-hexanediol instead of 1,5-pentanediol to give ethyl 9-hydroxy-3-oxanonanoate. This compound is then converted through a similar series of reactions to those described in Examples 1 and 2 as follows, the amount of material and yield of product being indicated in brackets (the other reactants specified in Examples 1 and 2 are used in the same molar proportion as in those examples with solvents being used in the same proportion of volume of solvent: weight of reactant).

| Reactant | Product |
|---|---|
| Ethyl 9-hydroxy-3-oxanonanoate (4 g) | Ethyl 8-formyl-3-oxaoctanoate (3.0 g, 77%) |
| Ethyl 8-formyl-3-oxaoctanoate (2.7 g) | Ethyl 11-oxo-9(E)-3-oxadodecanoate (2.2 g, 61%) |
| Ethyl 11-oxo-9(E)-3-oxadodecanoate (2.2 g) | 5-endo-(7'-Ethoxycarbonyl-6'-oxaheptyl)-6-exo-acetyl bicyclo[2,2,2] oct-2Z-ene and 5-exo-(7'-ethoxycarbonyl-6'-oxaheptyl)-6-endo-acetyl-bicyclo[2,2,2] oct-2Z-ene (1.8 g, 62%) |
| 5-endo-(7'-Ethoxycarbonyl-6'-oxaheptyl)-6-exo-acetyl bicyclo[2,2,2] oct-2Z-ene and 5-exo-(7'-ethoxycarbonyl-6-oxaheptyl)-6-endo-acetyl-bicyclo[2,2,2] oct-2Z-ene (1.8 g) | trans-5-(7'-Ethoxycarbonyl-6'-oxaheptyl)-6-acetyl-bicyclo[2,2,2] octane (1.7 g, 94%) |

| Reactant | Product |
|---|---|
| trans-5-(7'-Ethoxycarbonyl-6'-oxaheptyl)-6-acetyl-bicyclo[2,2,2] octane (1.7 g) | trans-5-(7'-Carboxy-6'-oxaheptyl)-6-acetyl-bicyclo[2,2,2] octane (1.4 g, 90%) |

The last mentioned product, which is the title compound, is obtained as an oil $\nu_{max}$ (film) 1730 (broad), 1705, 1125 $cm^{-1}$, δ($CDCl_3$) 9.55 (1H, br), 4.08 (2H, s), 3.52 (2H, t), 2.13 (3H, s), 2.15 (1H, m), 1.85 (1H, m), 1.75-1.1 (18H, m).

In a variation of this procedure the method used for the isolation of the product of the reaction of 1,6-hexane diol and ethyl bromoacetate may be varied as described in the variation of Example 1(1).

## Claims

1. A process for the preparation of a compound of formula (I)

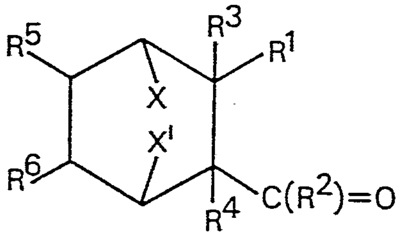

(I)

wherein X and X′ each represents hydrogen or together represent a bridging group $-CH_2-$, $-CH_2-CH_2-$ or -O-; $R^1$ is a 6-carboxyhex-2-enyl group or a modification thereof in which the group is altered by one, or an appropriate combination of two of more, of the following: (a) alteration of the position of the double bond, with the proviso that in the modified group the double bond is not in the alpha-beta position relative to the ring; (b) reduction of the double bond; (c) alteration of the chain length through a decrease of one or two methylene groups or an increase of one to six methylene groups; (d) replacement of one or two methylene groups each separately by an oxygen or sulphur atom, with the proviso that in the modified group no oxygen or sulphur atom is in an alpha position relative to either a doubly bonded carbon atom or to the carboxy group or a derivative thereof and that at least 2 carbon atoms separate any pair of oxygen and/or sulphur atoms; and (e) formation of an amide, ester or salt derivative of the carboxy group; $R^2$ is an aliphatic hydrocarbon group or an aliphatic hydrocarbon group substituted by an aromatic group directly or through an oxygen or sulphur atom; and $R^3$, $R^4$, $R^5$ and $R^6$ are each hydrogen, or $R^3$ and $R^4$ together and/or $R^5$ and $R^6$ together represent the second bond of a carbon-carbon double bond; which comprises effecting a Diels Alder reaction between a diene of formula (II) and a dienophile of formula (III)

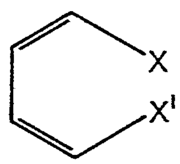

(II)

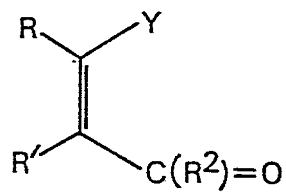

(III)

wherein X, X′ and $R^2$ are as defined for (I); Y is $R^1$ or a precursor therefor in which a double bond is present and/or the terminal group is in different form, either being a free carboxy group rather than the carboxy group derivative present in $R^1$ or being a carboxy group derivative rather than the free carboxy group present in $R^1$; and R and R′ are either each hydrogen or together represent the third bond of a carbon-carbon triple bond; and where appropriate thereafter, in either order, reducing the double bond or bonds in the ring of the Diels Alder adduct and/or converting the group Y to the group $R^1$.

2. A process according to Claim 1, in which $R^3$ and $R^4$, and R and R′, are each hydrogen.

3. A process according to Claim 1 or 2, in which $R^5$ and $R^6$ are each hydrogen, the Diels Alder adduct being reduced to provide the compound (I).

4. A process according to Claim 1, 2 or 3, in which X and X′ together represent a bridging group $-CH_2-$, $-CH_2-CH_2-$ or -O-.

5. A process for the preparation of a compound of formula (I)

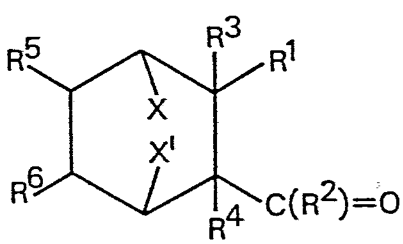

(I)

wherein X and X′ together represent a bridging group $-CH_2-CH_2-$; $R^1$ is a 6-carboxyhex-2-enyl group or a modification thereof in which the group is altered by one, or an appropriate combination of two of more, of the following: (a) alteration of the position of the double bond, with the proviso that in the modified group the double bond is not in the alpha-beta position relative to the ring; (b) reduction of the double bond; (c) alteration of the chain length through a decrease of one or two methylene groups or an increase of one to six methylene groups; (d) replacement of one or two methylene groups each separately by an oxygen or sulphur atom, with the proviso that in the modified group no oxygen or sulphur atom is in an alpha position relative to either a doubly bonded carbon atom or to the carboxy group or a derivative thereof and that at least 2 carbon atoms separate any pair of oxygen and/or sulphur atoms; and (e) formation of an amide, ester or salt derivative of the carboxy group; $R^2$ is an aliphatic hydrocarbon group or an aliphatic hydrocarbon group substituted by an aromatic group directly or through an oxygen or sulphur atom; and $R^3$, $R^4$, $R^5$ and $R^6$ are each hydrogen; which comprises effecting a Diels Alder reaction between a diene of formula (II) and a dienophile of formula (III)

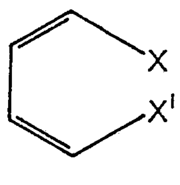

(II)

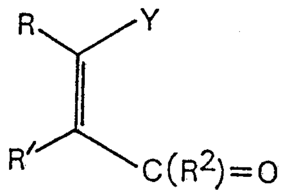

(III)

wherein X, X′ and $R^2$ are as defined for (I); Y is $R^1$ or a precursor therefor in which a double bond is present and/or the terminal group is in different form, either being a free carboxy group rather than the carboxy group derivative present in $R^1$ or being a carboxy group derivative rather than the free carboxy group present in $R^1$; and R and R′ are either each hydrogen or together represent the third bond of a carbon-carbon triple bond; and thereafter, in either order, reducing the double bond or bonds in the ring of the Diels Alder adduct and where appropriate converting the group Y to the group $R^1$.

6. A process according to any of Claims 1 to 5, in which the group $R^1$ incorporates a modification of type (b).

7. A process according to any of Claims 1 to 5, in which $R^1$ is $-(CH_2)_6-CO_2H$ or an amide, ester or salt derivative thereof.

8. A process according to any of Claims 1 to 6, in which the group $R^1$ incorporates a modification of type (d).

9. A process according to any of Claims 1 to 5, in which $R^1$ is a group $-(CH_2)_b-(A)_a-(Ch_2)_c-B-CH_2-CO_2H$ in which A and B are each separately oxygen or sulphur, a is 0, b is 0 and c is an integer from 3 to 10, or a is 1, b is 0 or an integer from 1 to 7 and c is an integer from 2 to 9 with the sum of b and c being from 2 to 9, or an amide, ester or salt derivative thereof.

10. A process according to Claim 9, in which $R^1$ is a group $(CH2)_b-(A)_a-(CH_2)_c-B-CH_2-CO_2H$ wherein a is 0, b is 0 and c is 4, 5 or 6, or a group $-(CH_2)_b-(A)_a-(CH_2)_c-B-CH_2-CO_2H$ wherein a is 1, b is 1, 2 or 3 and c is 2 or a is 1, b is 1 or 2 and c is 3, or an amide, ester or salt derivative thereof.

11. A process according to Claim 9 or 10, in which A and B are each oxygen.

12. A process according to Claims 1 to 6 and 8 to 11, in which any modification of $R^1$ of type (c) involves a decrease of one methylene group or an increase of one or two methylene groups.

13. A process according to any of Claims 1 to 5, in which $R^1$ is $-(CH_2)_4-O-CH_2-CO_2H$, $-(CH_2)_5-O-CH_2-CO_2H$, $-(CH_2)_6-O-CH_2-CO_2H$, $-CH_2-O-(CH_2)_2-O-CH_2-CO_2H$, $-CH_2-O-(CH_2)_3-O-CH_2-CO_2H$, $-(CH_2)_2-O-(CH_2)_2-O-CH_2-CO_2H$, $-(CH_2)_2-O-(CH_2)_3-O-CH_2-CO_2H$ or $-(CH_2)_3-O-(CH_2)_2-O-CH_2-CO_2H$, or an amide ester or salt derivative thereof.

14. A process according to any of the preceding claims, in which $R^2$ is hydrogen or an alkyl group or 1 to 3 carbon atoms.

15. A process according to Claims 14, in which $R^2$ is methyl.

16. A process according to Claim 1, in which the compound (I) contains a bicyclo [2,2,2] octane ring system having substituent $R^1$ which is a group $-(CH_2)_b-(A)_a-(CH_2)_c-B-CH_2-CO_2H$ wherein A and B are each oxygen, a is 0, bis 0 and c is 4, 5 or 6, or a is 1, b is 1, 2 or 3 and c is 2 or a is 1, b is 1 or 2 and c is 3, or an amide ester or salt derivative thereof, and a substituent $C(R^2)=0$ wherein $R^2$ is methyl or ethyl.

17. A process according to any of the preceding claims, in which the substituents $R^1$ and $C(R^2)=0$, and Y and $C(R^2)=0$, are in a trans relationship.

18. A process according to Claim 17, in which the divalent cyclic group of the compound (I) has the 5-endo, 6-exo configuration when it is a bicyclo [2,2,1] heptane, bicyclo [2,2,1] hept-2Z-ene or bicyclo [2,2,2] oct-2Z-ene, and the 5-endo, 6-exo or 5-exo, 6-endo configuration when it is a 7-oxa-bicyclo [2,2,1] heptane or 7-oxa-bicyclo [2,2,1] hept-2Z-ene.

19. A process according to Claim 1, in which the compound (I) contains a 5-endo, 6-exo-substituted bicyclo [2,2,1] heptane or a trans-5,6-substituted bicyclo [2,2,2] octane ring system wherein the

substituent at the 5-position is a 7-carboxy-6-oxaheptyl or 6-carboxy-5-oxahexyl group or an amide, ester or salt derivative thereof and the substituent at the 6-position is a grouping $C(R^2)=0$ in which $R^2$ is methyl or ethyl.

20. A process according to Claim 1, in which the compound (I) is 5-endo-(6′-carboxy-5′-oxahexyl)-6-exo-acetyl-bicyclo [2,2,1] heptane, 5-endo-(7′-carboxy-6′-oxaheptyl)-6-exo-acetyl-bicyclo [2,2,1] heptane, trans-5-(6′-carboxy-5′-oxahexyl)-6-acetyl-bicyclo [2,2,2] octane or trans-5-(7′-carboxy-6′-oxaheptyl)-6-acetyl-bicyclo [2,2,2] octane, or an ester thereof.

21. A process according to Claim 1, in which the compound (I) is 5-endo-(6′-carboxy-2′,5′-dioxahexyl)-6-exo-acetyl-bicyclo [2,2,1] heptane, 5-endo-(7′-carboxy-3′,6′-dioxaheptyl)-6-exo-acetyl-bicyclo [2,2,1] heptane, trans-5-(6′-carboxy-2′,5′-dioxahexyl)-6-acetyl-bicyclo [2,2,2] octane, trans-5-(7′-carboxy-3′,6′-dioxaheptyl)-6-acetyl-bicyclo [2,2,2] octane, or ester thereof.

22. A process according to any of the preceding claims, in which Y contains a carboxy group in derivative form.

23. A process according to any of the preceding claims, in which Y is either $R^1$ where $R^1$ is in amide or ester form or is an ester derivative of $R^1$ where $R^1$ is in free acid or salt form.

24. A process according to any of the preceding claims, in which the Diels Alder reaction is carried out in the presence of a Lewis acid catalyst.

25. A process according to Claim 24, in which the reaction is carried out in the presence of boron trifluoride.

26. A process according to any of Claims 8 to 11 and 13 as dependent on Claim 3 or 5, in which the reaction is effect by hydrogenation.

27. A process according to Claim 26, in which a ruthenium or rhodium catalyst is used.

28. A compound of formula (I) as defined in Claim 1, whenever prepared according to the process of any of Claims 1 to 27 or an obvious chemical equivalent thereof.